# EUROPÄISCHE PATENTANMELDUNG

(11) **EP 4 321 444 A1**
(43) Veröffentlichungstag der Anmeldung: **14.02.2024**
(21) Anmeldenummer: 22190269.5
(22) Anmeldetag: 12.08.2022
(51) Int. Cl.: B65B 55/02, A61J 7/00, A61M 5/178, B65B 59/00

(54) **VERFAHREN ZUR MONTAGE EINER ABGABEVORRICHTUNG EINES BEFÜLLTEN BEHÄLTERS, MONTIERVORRICHTUNG UND ABGABEVORRICHTUNG**

(71) Anmelder: TT Innovation AG, 6300 Zug (CH)
(72) Erfinder: Henzmann, Simon Daniel, 4052 Basel (CH); Huelin, Florian Simon, 5210 Windisch (CH); Maddalena, Tobias De, 4303 Kaiseraugust (CH)
(74) Vertreter: Mertzlufft-Paufler, Cornelius

(57) **Zusammenfassung**

Die Erfindung schlägt allgemein Verfahren zur Montage einer Abgabevorrichtung (2) eines befüllten Behälters (3) in einem vorzugsweise geschützten Arbeitsraum (5), wobei die Abgabevorrichtung (2) aus wenigstens einem ersten Geräteteil (6), insbesondere einem zu einem Auslösen einer Abgabe eines Behälterinhalts aus dem Behälter (3) eingerichteten Betätigerteil (7), und einem zweiten Geräteteil (9), vorzugsweise einem befüllten Behälterkörper (10), montiert wird, vor, umfassend folgende Verfahrensschritte: Durchströmung des Arbeitsraums (5) mit einer vorzugsweise laminaren und/oder unidirektionalen Luftströmung (4); Positionierung des ersten und zweiten Geräteteils (6, 9) in der Luftströmung (4), und Verbinden des ersten Geräteteils (6) mit dem zweiten Geräteteil (9) abströmseitig des zweiten Geräteteils (9)

## Beschreibung

Die Erfindung betrifft ein Verfahren zur Montage einer Abgabevorrichtung eines befüllten Behälters in einem vorzugsweise geschützten Arbeitsraum, wobei die Abgabevorrichtung aus wenigstens einem ersten Geräteteil, insbesondere einem zu einem Auslösen einer Abgabe eines Behälterinhalts aus dem Behälter eingerichteten Betätigerteil, und einem zweiten Geräteteil, vorzugsweise einem befüllten Behälterkörper, montiert wird.

Derartige Verfahren werden insbesondere verwendet zur Montage von pharmazeutischen Geräten und/oder Medizinprodukten, beispielsweise Injektoren wie Autoinjektoren oder Sicherheitsspritzen. Es ist üblich, dass derartige Verfahren häufig in geschützten Arbeitsräumen durchgeführt werden. Unter geschützten Arbeitsräumen sind insbesondere Isolatoren und/oder RABS (Restricted Access Barrier System) zu verstehen. Geschützte Arbeitsräume werden oftmals mit einer Lüftungsanlage betrieben, sodass eine optimale Luftströmung im geschützten Arbeitsraum aufrechterhalten werden kann. Eine Luftströmung kann insbesondere eine Verdrängungsströmung (laminar) sein und/oder eine Mischströmung (turbulent). Durch eine Luftströmung kann gewährleistet werden, dass die zu montierenden Geräte partikel- und/oder kontaminationsfrei montiert werden. Bei Verfahren bekannter Art wird oftmals ein erstes Geräteteil in einer Luftströmung zuströmseitig des zweiten Geräteteils verbunden. Beispielsweise werden bei Sicherheitsspritzen Kolbenstange (Plunger Rods) zuströmseitig des Spritzenkörpers in den Spritzenkörper geschraubt. Nachteilig hieran ist, dass die Handhabevorrichtung(en), welche zur Montage des ersten und des zweiten Geräteteils verwendet wird/werden, oberhalb, also beispielsweise zuströmseitig des ersten Geräteteils aufgebaut und/oder positioniert wird/werden. Somit muss bei einer Montiervorrichtung genügend Arbeitsraum oberhalb einer Montageposition einer Montage der Abgabevorrichtung vorhanden sein. Ferner von Nachteil kann sein, dass eine Aufrechterhaltung der Luftströmung besonders aufwendig und kostenintensiv sein kann. Dies kann insbesondere daran liegen, dass, wie zuvor erwähnt, Handhabevorrichtungen zuströmseitig der zu montierenden Geräteteile in der Luftströmung positioniert werden, wodurch die Luftströmungen bereits aufgrund von Umlenkungen aufwendig korrigiert werden müssen.

Die Erfindung betrifft ferner eine Montiervorrichtung zur Montage einer Abgabevorrichtung eines befüllten Behälters in einem vorzugsweise geschützten, mit einer Luftströmung durchströmten Arbeitsraum, wobei die Abgabevorrichtung wenigstens ein erstes Geräteteil, insbesondere ein zu einem Auslösen einer Abgabe eines Behälterinhalts aus dem Behälter eingerichtetes Betätigerteil, und ein zweites Geräteteil, insbesondere einen befüllten Behälterkörper, umfasst. Derartige Montiervorrichtungen sind aus dem Stand der Technik bekannt und sind häufig konstruktiv aufwändig, da wie bereits zuvor beschrieben, Arbeitsräume aufgrund der Handhabevorrichtungen hoch ausgebildet sind und die Aufrechterhaltung von Luftströmungen kostenintensiv ist.

Die Erfindung betritt schließlich eine Abgabevorrichtung.

Es ist daher Aufgabe der Erfindung Montagen einer Abgabevorrichtung eines befüllten Behälters in einem vorzugsweise geschützten Arbeitsraum zu verbessern.

Zur Lösung der genannten Aufgabe sind erfindungsgemäß die Merkmale des nebengeordneten, auf ein Verfahren gerichteten Anspruchs vorgesehen. Insbesondere wird somit zur Lösung der genannten Aufgabe bei einem Verfahren der eingangs beschriebenen Art erfindungsgemäß vorgeschlagen, dass das Verfahren folgende Verfahrensschritte umfasst: Durchströmung des Arbeitsraums mit einer vorzugsweise lokal eine Hauptströmung definierenden Luftströmung; Positionierung des ersten und zweiten Geräteteils in der Luftströmung, und Verbinden des ersten Geräteteils mit dem zweiten Geräteteil abströmseitig des zweiten Geräteteils. Hierdurch werden das erste und zweite Geräteteil in der Luftströmung miteinander verbunden, wobei vorteilhaft das erstes Geräteteil abströmseitig, also im Windschatten des zweiten Geräteteils, mit dem zweiten Geräteteil verbunden wird. Hierdurch kann ein Verbindungsegment des zweiten Geräteteils zumindest während des Verbindens des ersten und zweiten Geräteteils frei von Luftdurchwirbelungen der Luftströmung sein. Somit kann ein Eintrag von Partikeln vorzugsweise in dem befüllten Bereich des Behälterkörpers vermindert oder gar verhindert werden, was insbesondere für nachfolgende Applikationen der Abgabevorrichtung von Vorteil sein kann. Ebenso kann ein konstruktiver Aufbau einer Montiervorrichtung, welche zur Durchführung des Verfahrens geeignet ist und verwendet wird, insbesondere die hierin beschriebene Montiervorrichtung, vereinfacht und kostengünstiger ausgebildet werden. Das erfindungsgemäße Verfahren ist somit verbessert.

Durch die abströmseitige Positionierung im Windschatten des zweiten Geräteteils kann auf einfache Weise erreicht werden, dass eine Vorrichtung zum Verbinden, insbesondere Verschrauben, die Luftströmung auf der Anströmseite nicht stört und dort somit definierte Strömungsverhältnisse erreichbar sind. Eine Luftströmung kann insbesondere eine gerichtete Verdrängungsströmung, beispielsweise laminar oder eine turbulente Mischströmung sein, insbesondere wie es für die hierin beschriebenen Isolatoren und RABS und Reinräumen dem Fachmann bekannt ist.

Eine Positionierung des ersten und zweiten Geräteteils kann durch eine oder mehrere Vorrichtungen, insbesondere der hierin genannten Handhabevorrichtungen und/oder Geräteaufnahme(n) durchgeführt werden. Durch die Wahl der spezifischen Vorrichtung, insbesondere durch die Wahl der Anzahl an Vorrichtungen, kann eine Taktzahl an zu montierenden Abgabevorrichtungen erhöht werden. Somit können durch ein erfindungsgemäßes Verfahren effizient große Stückzahlen produziert werden. Dies ist insbesondere von Vorteil, wenn die Abgabevorrichtung ein Medizingerät zur einmaligen Nutzung ist, jedoch die Art der Abgabevorrichtung häufig benutzt wird. Dies ist beispielsweise der Fall bei Sicherheitsspritzen, welche bevorzugt durch das erfindungsgemäße Verfahren und die hierin beschriebene Montiervorrichtung hergestellt werden.

Unter Verbinden ist jede dem Fachmann bekannt Kopplung von zwei Geräteteilen zu verstehen. Beispielsweise kann ein erstes Geräteteil abströmseitig des zweiten Geräteteils in das zweite Geräteteil gesteckt werden. Insbesondere ist ein wie hierin beschriebenes Verschrauben als Verbinden zu verstehen. Je nach zu montierender Abgabevorrichtung kann durch die zuvor genannte Vielfalt an Verbindungsmöglichkeiten vorteilhaft eine Verbindungsmöglichkeit verwendet werden.

Bei einer vorteilhaften Ausgestaltung kann vorgesehen sein, dass das erste Geräteteil eine Kolbenstange (Plunger Rod) hat und/oder das zweite Geräteteil einen Spritzenkörper hat. Somit kann vorteilhaft ein Sicherheitsspritze durch das Verfahren hergestellt werden, wobei vorteilhaft die Kolbenstange abströmseitig des Spritzenkörpers mit dem Spritzenkörper verbunden wird. Typischerweise wird die Kolbenstange in den Spritzenkörper eingeschraubt (oder auf diesen aufgeschraubt) und nicht umgekehrt, so dass in diesem Fall ein Antrieb für die Schraubbewegung im Windschatten der Luftströmung angeordnet werden kann und diese nicht auf Höhe der Geräteteile stört.

Bei einer vorteilhaften Ausgestaltung kann vorgesehen sein, dass das erste Geräteteil mit einem beweglichen Stopfen des zweiten Geräteteils verbunden wird. Dies ist insbesondere dann von Vorteil, wenn das erste Geräteteil, wie zuvor beschrieben eine Kolbenstange ist, und das zweite Geräteteil, wie zuvor beschrieben einen befüllten Spritzenkörper hat. Vorteilhaft daran kann nämlich sein, dass durch die Haftreibung des beweglichen Stopfens und durch Gegendruck an dem Spritzenkörper verhindert wird, dass Flüssigkeit aus dem Spritzenkörper spritzt. Somit kann insbesondere bei einer Sicherheitsspritze die von der Nadel der Sicherheitsspritze abgewandte Seite abströmseitig positioniert werden.

Bei einer vorteilhaften Ausgestaltung kann vorgesehen sein, dass eine Handhabevorrichtung das erste und/oder das zweite Geräteteil aus einem Lager entnimmt, wobei die Handhabevorrichtung zur Positionierung des ersten Geräteteils das erste Geräteteil an eine Geräteaufnahme überführt. Somit kann vorteilhaft das erste Geräteteil an die Geräteaufnahme überführt werden, um anschließend durch die Geräteaufnahme in der Luftströmung und abströmseitig des zweiten Geräteteils positioniert werden zu können.

Insbesondere kann als erste Handhabevorrichtung ein Greifroboter vorgesehen sein. Somit kann eine Positionierung des ersten und/oder zweiten Geräteteils besonders variabel, beispielsweise variabel in der Anzahl der Freiheitsgrade, ausgeführt werden.

Alternativ oder zusätzlich kann vorgesehen sein, dass die Handhabevorrichtung zur Positionierung des zweiten Geräteteils das zweite Geräteteil an eine zweite Handhabevorrichtung, insbesondere einem zweiten Greifroboter, übergibt. Somit kann vorteilhaft das zweite Geräteteil an eine zweite Handhabevorrichtung übergeben werden. Diese Ausführungsform ist besonders vorteilhaft, da hierdurch eine Montage des ersten und zweiten Geräteteils durch die hierin beschriebene Geräteaufnahme und die zweite Handhabevorrichtung durchgeführt werden kann. Während der Montage kann die erste Handhabevorrichtung ein weiteres Geräteteil greifen, welche für eine anschließende Montage einer Abgabevorrichtung bereitgestellt werden kann. Somit kann eine Gesamtprozesslaufzeit von Montagen kürzer sein.

Bei einer vorteilhaften Ausgestaltung kann vorgesehen sein, dass das erstes Geräteteil abströmseitig des zweiten Geräteteils eingeführt, insbesondere eingeschraubt, wird. Somit kann das erste Geräteteil, beispielsweise durch die bereits erwähnte Geräteaufnahme, vorteilhaft durch eine Bewegung an der abströmseitigen Seite des zweiten Geräteteils eingeführt werden. Insbesondere kann ein Einschrauben des ersten Geräteteils in das zweite Geräteteil vorgesehen sein. Besonders vorteilhaft an dieser Ausführungsform mit einer besonders hohen erfinderischen Tragweite, kann sein, dass die Montage des ersten und zweiten Geräteteils so durchgeführt werden kann, dass oberhalb des zweiten Geräteteils eine freie Zone zum Arbeiten anderer Vorrichtungen, beispielsweise Handhabeverrichtungen, vorgesehen sein kann, da zum Einführen bzw. Einschrauben keinerlei Platz oberhalb des zweiten Geräteteils benötigt wird.

Vorzugsweise kann vorgesehen sein, dass das Einführen, insbesondere Einschrauben, des ersten Geräteteils durch eine der Luftströmung entgegengerichteten Hubbewegung der Geräteaufnahme durchgeführt wird.

Insbesondere kann vorgesehen sein, dass die Geräteaufnahme einen Dreh-Hubbewegung durchführt. Somit kann vorteilhaft das erste Geräteteil in das zweite Geräteteil eingeschraubt werden.

Bei einer vorteilhaften Ausgestaltung kann vorgesehen sein, dass ein Drehmoment und/oder ein Anpressdruck während des abströmseitigen Verbindens insbesondere durch die Geräteaufnahme überwacht wird. Somit kann vorteilhaft verhindert werden, dass das Drehmoment und/oder der Anpressdruck während des Verbindens so groß wird, dass die in dem zweiten Geräteteil befindliche Flüssigkeit aus dem zweiten Geräteteil entweicht.

Bei einer vorteilhaften Ausgestaltung kann vorgesehen sein, dass das erste Geräteteil durch die Geräteaufnahme lösbar gegriffen wird. Hierdurch kann das erste Geräteteil und/oder eine montierte Abgabevorrichtung nach erfolgter Montage leicht von der Geräteaufnahme gelöst und von dieser entfernt werden.

Dies ist insbesondere von Vorteil, wenn, wie vorzugsweise vorgesehen ist, das erste Geräteteil durch die Geräteaufnahme an seinem Kopfende gegriffen wird. Durch das Greifen des ersten Geräteteils an dessen Kopfende kann vorteilhaft genügend Platz des ersten Geräteteils zum Einführen, insbesondere Einschrauben, erhalten bleiben. Insbesondere von Vorteil ist diese Ausführungsform, wenn eine Kolbenstange in eine Spritzenkörper eingeschraubt wird.

Bei einer vorteilhaften Ausgestaltung kann vorgesehen sein, dass das zweite Geräteteil durch die erste oder zweite Handhabevorrichtung zuströmseitig des ersten Geräteteils positioniert wird. Somit kann das zweite Geräteteil vorteilhaft in der Luftströmung beispielsweise oberhalb des ersten Geräteteils und/oder der hierin beschriebenen Geräteaufnahme positioniert werden. Insbesondere von Vorteil daran kann sein, dass das zweite Geräteteil durch die zweite Handhabevorrichtung zuströmseitig gehalten wird, während das erste Geräteteil durch die erste Handhabevorrichtung oder durch die Geräteaufnahme gehalten wird. Somit kann ein Gesamtprozess besonders schnell durchgeführt werden.

Alternativ oder zusätzlich kann vorgesehen sein, dass das zweite Geräteteil durch die erste oder zweite Handhabevorrichtung gehalten und zur Positionierung ausgerichtet wird. Insbesondere kann vorgesehen sein, dass das zweite Geräteteil durch die erste oder zweite Handhabevorrichtung in einem Winkel beispielsweise von 90° oder 180° gedreht wird, um vorteilhaft zur Montage positioniert zu werden. Der Drehwinkel kann beispielsweise in Bezug auf die Strömungsrichtung definiert sein. Durch eine Drehung des zweiten Geräteteils kann ein Verbindungselement des zweiten Geräteteils abströmseitig positioniert werden. Somit kann das zweite Geräteteil auch liegend, überkopf oder, insbesondere bei einer hierin beschriebenen Sicherheitsspritze, mit einer Nadel abströmseitig positioniert werden.

Bei einer vorteilhaften Ausgestaltung kann vorgesehen sein, dass vorzugsweise eine Eigenschaft/Merkmal, insbesondere eine Ausrichtung und/oder Farbe, des ersten und/oder des zweiten Geräteteils inspiziert wird. Somit kann vorteilhaft verhindert werden, dass unpassende und/oder falsch ausgerichtete Geräteteile miteinander verbunden werden. Somit können Fehlmontagen und/oder Prozessstopps vorteilhaft vermieden werden.

Insbesondere kann vorgesehen sein, dass die Inspektion stattfindet, während das erste und/oder das zweite Geräteteil durch die erste Handhabevorrichtung gegriffen wird/werden. Somit kann besonders schnell eine Inspektion durchgeführt werden. Weiterhin kann vorteilhaft ermöglicht werden, dass ein als falsch ausgerichtetes und durch die Inspektion als solches identifiziertes Geräteteil durch die Handhabevorrichtung zurück in das beispielsweise zuvor genannte Lager überführt werden kann. Somit kann insgesamt eine Gesamtprozesslaufzeit aufrechterhalten werden, ohne großartige Verzögerungen in Kauf nehmen zu müssen. Ferner kann eine Beschädigung von Handhabevorrichtungen und/oder Geräteaufnahmen, insbesondere die hierin beschriebenen, verhindert werden, welche durch Montage fehlerhaft ausgerichteter Geräteteil entstehen könnten.

Bei einer vorteilhaften Ausgestaltung kann vorgesehen sein, dass die erste und/oder die zweite Handhabevorrichtung nach dem Einführen eine Aufwärtshubbewegung durchführen. Somit kann die Abgabevorrichtung nach erfolgter Montage des ersten und zweiten Geräteteils aus der Montageposition entfernt werden, sodass ein Materialfluss aufrechterhalten bleiben kann. So kann, wie vorzugsweise vorgesehen, die Abgabevorrichtung durch die erste und/oder zweite Handhabevorrichtung vorteilhaft in ein Formteil überführt werden. Dabei kann das Formteil passgenau an ein drittes Gerätteil, beispielsweise ein wie hierin beschriebenes Case, angepasst sein. Somit kann vorteilhaft die Abgabevorrichtung durch die Überführung in ein Formteil final mit dem dritten Geräteteil montiert werden.

Es kann alternativ oder zusätzlich vorgesehen sein, dass die Abgabevorrichtung in ein Lager übergeben wird. Somit kann die montierte Abgabevorrichtung direkt nach der Montage gelagert werden. Insbesondere von Vorteil ist diese Ausführungsform, wenn das Lager, wie vorgesehen sein kann und nachfolgend beschrieben ist, ein passgenaues Formteil und/oder ein drittes Geräteteil hat. Hierdurch kann eine Montage besonders schnell durchgeführt werden.

Es kann auch alternativ oder zusätzlich vorgesehen sein, dass die Abgabevorrichtung in das zuvor genannte dritte Geräteteil (Case) überführt wird. Somit kann eine finale Montage der Sicherheitsspritze besonders schnell durchgeführt werden.

Alternativ oder zusätzlich kann die Abgabevorrichtung durch die erste und/oder zweite Handhabevorrichtung auf ein Transportsystem überführt werden. Somit kann die Abgabevorrichtung weiter transportiert werden, wodurch ein Gesamtprozess besonders schnell durchgeführt werden kann.

Es kann insbesondere vorgesehen sein, dass das Transportsystem eine magnetisch levitierte Transporteinheit umfasst. Somit kann vorteilhaft die Abgabevorrichtung auf eine magnetisch levitierte Transporteinheit überführt und durch diese weitertransportiert werden. Durch die magnetische Levitation der Transporteinheit kann ein Transport der Abgabevorrichtung reibungslos durchgeführt werden, was besonders in geschützten Arbeitsräumen bevorzugt ist.

Alternativ oder zusätzlich sind zur Lösung der genannten Aufgabe erfindungsgemäß die Merkmale des nebengeordneten, auf eine Montiervorrichtung gerichteten Anspruchs vorgesehen. Insbesondere wird somit zur Lösung der genannten Aufgabe bei einer Montiervorrichtung der eingangs beschriebenen Art erfindungsgemäß vorgeschlagen, dass die Montiervorrichtung umfasst: eine Geräteaufnahme zur Positionierung des ersten Geräteteils; wenigstens ein Handhabevorrichtung zur Positionierung des zweiten Geräteteils zuströmseitig des ersten Geräteteils; wenigstens eine Verbindungsvorrichtung, insbesondere Verschraubvorrichtung, zum Verbinden, insbesondere Verschrauben, des erste Geräteteils mit dem zweiten Geräteteil. Vorteilhaft daran ist die Eignung der Montiervorrichtung zur Durchführung eines wie zuvor beschriebenen bzw. nachfolgend beanspruchten Verfahrens zur Montage einer Abgabevorrichtung eines befüllten Behälters in einem vorzugsweise geschützten Arbeitsraum. Die Durchführung eines wie hierin beschriebenen Verfahrens ist auch insbesondere vorgesehen.

Ein geschützter Arbeitsraum kann beispielsweise ein Isolator oder ein RABS sein, wodurch die Montiervorrichtung besonders geeignet ist pharmazeutische Geräte und/oder Medizinprodukte, beispielsweise die zuvor genannten Sicherheitsspritzen, herzustellen. Die Luftströmung kann durch den Fachmann bekannte Ventilatoren, Zu- und Abluftöffnungen aufgebaut bzw. aufrechterhalten werden.

Die Geräteaufnahme kann insbesondere das erste Geräteteil, welches vorzugsweise ein zu einem Auslösen einer Abgabe eines Behälterinhalts aus dem Behälter eingerichtetes Betätigerteil umfasst, greifen und in der Luftströmung positionieren. Die Geräteaufnahme kann insbesondere so positioniert sein, dass sie sich während der Montage des ersten und zweiten Geräteteils abströmseitig des ersten und/oder zweiten Geräteteils befindet. Hierdurch kann vorteilhaft ein schmaler, vorzugsweise vertikaler Montagekorridor geschaffen sein. Somit kann insbesondere ein Volumen eines geschützten Arbeitsraums optimal ausgenutzt werden.

Durch die wenigstens eine Handhabevorrichtung kann vorteilhaft das zweite Geräteteil zuströmseitig des ersten Geräteteils positioniert werden. Die Handhabevorrichtung kann aber auch, insbesondere wie nachfolgend beschrieben und beansprucht, das erste Geräteteil greifen und/oder an die Geräteaufnahme übergeben. Eine Handhabevorrichtung ist vielfältig einsetzbar und kann vorzugsweise mehrerer Teilschritte einer Montage ausführen. Somit kann die Montiervorrichtung besonders flexibel ausgebildet sein. Eine Flexibilität der Montiervorrichtung ist auch daher ableitbar, dass die Montiervorrichtung zur Montage bautyp-verschiedener Abgabevorrichtungen geeignet ist.

Die Verbindungsvorrichtung kann das erste Geräteteil abströmseitig des zweiten Geräteteils mit dem zweiten Geräteteil verbinden. Somit wird durch die Montiervorrichtung eine Montage der Abgabevorrichtung im Windschatten des zweiten Geräteteils durchgeführt, wodurch konstruktiv mehr Raum und Flexibilität geschaffen werden kann. Dies ist insbesondere vorteilhaft in geschützten Arbeitsräumen, wie die bereits erwähnten Isolatoren und RABS.

Die Verbindungsvorrichtung kann unterschiedlich und fachmännisch bekannt ausgebildet sein, um das erste und zweite Geräteteil abströmseitig des zweiten Geräteteils zu verbinden. Besonders vorteilhaft ist jedoch vorgesehen, dass die Verbindungsvorrichtung als Verschraubvorrichtung zum Einschrauben des ersten Geräteteils mit dem zweiten Geräteteil ausgebildet ist. Ein Einschrauben ist, wie die Erfindung erkannt hat, insbesondere vorteilhaft, wenn wie nachfolgend beschrieben und beansprucht, das erste Geräteteil eine Kolbenstange (Plunger Rod) hat und das zweite Geräteteil einen Spritzenkörper. Somit können besonders vorteilhaft Sicherheitsspritzen durch die Montiervorrichtung, insbesondere bei einem hierin beschriebenen Verfahren, montiert werden. Insgesamt kann ein Montiervorrichtung eine Montage einer Abgabevorrichtung verbessern.

Bei einer vorteilhaften Ausgestaltung einer Montiervorrichtung kann vorgesehen sein, dass das erste Geräteteil eine Kolbenstange (Plunger Rod) hat und/oder das zweite Geräteteil einen Spritzenkörper. Somit kann die Montiervorrichtung vorteilhaft Sicherheitsspritzen montieren.

Bei einer vorteilhaften Ausgestaltung einer Montiervorrichtung kann vorgesehen sein, dass das erste Geräteteil mit einem beweglichen Stopfen des Behälters verbindbar ist. Der bewegliche Stopfen ist so an den Behälter gekoppelt, dass während des Verfahrens der Montage der Abgabevorrichtung keine Flüssigkeit austreten kann. Somit kann der befüllte Behälter beispielsweise, wie zuvor beschrieben, in einem Winkel von 180° gedreht werden, ohne dass die Flüssigkeit aus dem Behälter entweicht.

Der bewegliche Stopfen ist unter anderem für eine kraft- und formschlüssige Verbindung mit dem ersten Geräteteil ausgebildet. Dabei muss das eingebrachte Verbindungsdrehmoment unterhalb des Haftdrehmoments des Stopfens zur Spritze liegen, sodass der Stopfen bei der Verbindung nicht bewegt wird.

Bei einer vorteilhaften Ausgestaltung einer Montiervorrichtung kann vorgesehen sein, dass die Geräteaufnahme die Verbindungsvorrichtung ist. Somit kann die Positionierung des ersten Geräteteils und das Verbinden des ersten Geräteteils mit dem zweiten Geräteteil durch dieselbe Vorrichtung ausgeführt werden. Somit können bei der Montiervorrichtung Material und Kosten eingespart werden.

Insbesondere kann vorgesehen sein, dass die Geräteaufnahme zum Verbinden des ersten Geräteteils in das zweite Geräteteil entgegen der Luftströmung hubbewegbar ist. Somit kann insbesondere eine wie hierin beschriebene Hubbewegung, vorzugweise im Verlauf einer Z-Achse, durchgeführt werden. Diese Ausführungsform zeugt von einer besonders hohen erfinderischen Idee und ist besonders vorteilhaft, da eine Vorrichtung bzw. ein Kraftaufbringen zum Verbinden des ersten und zweiten Geräteteils unterhalb, und nicht oberhalb, des ersten und zweiten Geräteteils positioniert werden kann. Somit kann sich vorteilhaft eine Vorrichtung, beispielsweise eine hierin genannte Handhabevorrichtung, frei zuströmseitig des zweiten Geräteteils bewegen. Durch einen Freiraum zuströmseitig des zweiten Geräteteils kann eine Luftströmung im geschützten Arbeitsraum einfacher aufrechterhalten werden.

Eine Geräteaufnahme kann auch dreh-hub-bewegbar sein, wodurch das erste Geräteteil mit dem zweiten Geräteteil abströmseitig des zweiten Geräteteils verschraubt werden kann. Ferner kann somit insbesondere eine wie hierin beschriebene Drehhubbewegung, insbesondere im Verlauf der genannten Z-Achse, durchgeführt werden.

Bei einer vorteilhaften Ausgestaltung einer Montiervorrichtung kann vorgesehen sein, dass die Geräteaufnahme ein oder maximal zwei Freiheitsgrade hat. Hierdurch kann die Geräteaufnahme konstruktiv einfach und kostengünstig ausgebildet werden. Auf eine vorzugsweise wie hierin beschriebene Handhabevorrichtung kann als Verbindungsvorrichtung verzichtet werden. Eine insbesondere nach dieser Ausgestaltung ausgebildete Geräteaufnahme kann beispielsweise hubbewegbar und/oder drehhubbewegbar sein, um ein Verbinden bzw. Einschrauben einer Abgabevorrichtung zu ermöglichen.

Bei einer vorteilhaften Ausgestaltung einer Montiervorrichtung kann vorgesehen sein, dass das erste Geräteteil durch die wenigstens eine Handhabevorrichtung an die Geräteaufnahme überführbar ist. Somit kann vorteilhaft das erste Geräteteil durch die Handhabevorrichtung auf die Geräteaufnahme überführt werden.

Bei einer vorteilhaften Ausgestaltung einer Montiervorrichtung kann vorgesehen sein, dass die Gerätaufnahme eine Greifvorrichtung aufweist. Hierdurch kann vorteilhaft das erste Geräteteil gegriffen werden. Durch ein Greifen kann das erste Geräteteil insbesondere bei dem Verbinden bzw. Einschrauben des ersten und zweiten Geräteteils fixiert sein, wodurch Abgabevorrichtungen fehlerfrei montiert werden können.

Insbesondere ist die Greifvorrichtung eine Dreh-Hub-Vorrichtung. Eine als Dreh-Hub-Vorrichtung ausgebildete Greifvorrichtung kann vorteilhaft zum Einschrauben des ersten Geräteteils mit dem zweiten Geräteteil Verwendung finden.

Bei alternativen Ausführungsformen kann die Geräteaufnahme andere, dem Fachmann bekannten Vorrichtungen haben, an welche das erste Geräteteil übergeben werden kann. Beispielsweise kann die Geräteaufnahme eine Auflage ausbilden. Eine Geräteaufnahme kann somit an eine Ausgestaltung des ersten Geräteteils angepasst sein. Somit kann durch eine Montiervorrichtung Abgabevorrichtungen unterschiedlichen Bautyps hergestellt werden, was besonders kundenfreundlich sein kann.

Bei einer vorteilhaften Ausgestaltung einer Montiervorrichtung kann vorgesehen sein, dass die Geräteaufnahme zur Überwachung eines Drehmoments und/oder eines Anpressdrucks eingerichtet ist. Somit kann die Geräteaufnahme ein Drehmoment und/oder ein Anpressdruck während des Verbindens des ersten Geräteteils mit dem zweiten Geräteteil überwachen. Hierdurch kann vorteilhaft eine fehlerhafte Montage einer Abgabevorrichtungen verhindert werden. Somit kann insgesamt eine Montagequalität, insbesondere in Serienproduktion, verbessert werden.

Bei einer vorteilhaften Ausgestaltung einer Montiervorrichtung kann vorgesehen sein, dass die Greifvorrichtung das erste Geräteteil an einem Kopfende lösbar greift. Somit kann vorteilhaft der nicht durch die Greifvorrichtung gegriffenen Teil frei und abströmseitig des zweiten Geräteteils positioniert und verbunden werden. Insgesamt kann das Greifen am Kopfende eine größere Flexibilität bezüglich der Ausbildung des ersten Geräteteils, insbesondere einem Verbindungsbereich zum Verbinden mit dem zweiten Geräteteil, ermöglichen. Durch das lösbare Greifen kann das erste Geräteteil, insbesondere nach erfolgter Montage, freigegeben werden.

Bei einer vorteilhaften Ausgestaltung einer Montiervorrichtung kann vorgesehen sein, dass die Montiervorrichtung wenigstens eine erste und eine zweite Handhabevorrichtung aufweist, wobei wenigstens ein Geräteteil durch die erste Handhabevorrichtung an die zweite Handhabevorrichtung übergebbar ist. Somit kann ein Geräteteil von der ersten auf die zweite Handhabevorrichtung übergeben werden. Vorzugsweise wird das zweite Geräteteil übergeben. Somit kann ein Verbinden der Geräteteile der Abgabevorrichtung durch die zweite Handhabevorrichtung, welche das zweite Geräteteil positioniert, und die Geräteaufnahme, welche das erste Geräteteil positioniert, durchgeführt werden. Während des Verbindens des ersten und zweiten Geräteteils kann die erste Handhabevorrichtung ein weiteres Geräteteil, zur Montage einer weiteren Abgabevorrichtung, greifen. Somit kann durch die Verwendung von wenigstens zwei Handhabevorrichtungen eine größere Stückzahl an Abgabevorrichtungen produziert werden, was besonders vorteilhaft ist.

Bei einer vorteilhaften Ausgestaltung einer Montiervorrichtung kann vorgesehen sein, dass das erste und/oder das zweite Geräteteil und/oder die montierte Abgabevorrichtung durch die erste und/oder zweite Handhabevorrichtung in wenigstens drei Freiheitsgraden bewegbar und/oder positionierbar ist. Somit kann eine Bewegung und/oder Positionierung von Geräteilen und/oder montierten Abgabevorrichtungen besonders flexibel ausgestaltet werden. Insbesondere ist dies vorteilhaft, wenn wie hierin beschrieben, die Geräteaufnahme die Verbindungsvorrichtung ist, da der hierdurch erzielte Freiraum zuströmseitig des in der Luftströmung positionierten zweiten Geräteteils, weiter freigehalten und/oder für weitere Prozessschritte Verwendung finden kann.

Bei einer vorteilhaften Ausgestaltung einer Montiervorrichtung kann vorgesehen sein, dass die Montiervorrichtung eine Inspektionsvorrichtung, insbesondere zur Inspektion einer Eigenschaft des ersten und/oder zweiten Geräteteils aufweist. Somit kann das erste und/oder zweite Geräteteil inspiziert werden. Hierdurch kann vorteilhaft eine Identifizierung eines Geräteteils durchgeführt werden. Somit kann verhindert werden, dass Geräteteile, welche zu unterschiedlichen Bautypen von Abgabevorrichtungen gehören, montiert werden. Dies ist insbesondere von Vorteil in der Massenproduktion und vorzugsweise dann, wenn, wie vorgesehen sein kann, Abgabevorrichtungen unterschiedlichen Bautyps in einer Montiervorrichtung montiert werden.

Als Eigenschaft kann insbesondere eine Ausrichtung und/oder eine Farbe des ersten und/oder zweiten Geräteteils dienen.

Bei einer vorteilhaften Ausgestaltung einer Montiervorrichtung kann vorgesehen sein, dass der geschützte Arbeitsraum eine Lüftungsanlage und/oder Luftöffnungen zur Ausbildung der Luftströmung aufweist. Durch die Lüftungsanlage kann vorteilhaft und insbesondere eine wie hierin beschriebene Luftströmung im geschützten Arbeitsraum erzeugt und/oder aufrechterhalten werden.

Bei einer vorteilhaften Ausgestaltung einer Montiervorrichtung kann vorgesehen sein, dass die montierte Abgabevorrichtung durch die erste und/oder zweite Handhabevorrichtung an ein passgenaues Formteil übergebbar ist. Hierdurch kann ein Abgabevorrichtung sicher an ein passgenaues Formteil übergeben werden, wodurch ein Materialfluss weitergeführt und ein Produktionsstau verhindert werden kann. Das Formteil ist vorzugsweise passgenau an ein Geräteteil, vorzugsweise ein wie nachfolgend beschriebenes und beanspruchtes drittes Geräteteil, angepasst.

Vorzugsweise befindet sich das Formteil auf einer vorzugsweise magnetisch levitierbaren Transporteinheit. Somit kann die in ein passgenaues Formteil übergebene Abgabevorrichtung reibungslos und partikelfrei nach erfolgter Übergabe weitertransportiert werden. Die Transporteinheit kann als Teil des zuvor beschriebenen Transportsystems ausgebildet sein, wobei das Transportsystem zumindest eine magnetische StatorAnordnung umfasst, an welche die Transporteinheit magnetisch koppelbar ist und zumindest während eines Verfahrens der Transporteinheit gekoppelt wird.

Insbesondere kann vorgesehen sein, dass sich an dem passgenauen Formteil ein drittes Geräteteil (Case) befindet, in welches die Abgabevorrichtung eingesetzt wird. Das dritte Geräteteil kann in einer besonders bevorzugten Ausführungsform ein Case einer Sicherheitsspritze sein. Das Case kann einoder mehrteilig ausgebildet sein. Insbesondere bei dieser Ausführungsform kann die Sicherheitsspritze final montiert und beispielsweise durch die hierin beschriebene, levitierende Transporteinheit abtransportiert werden.

Alternativ oder zusätzlich kann vorgesehen sein, dass das passgenaue Formteil in einem Lager positioniert ist. Somit kann die montierte Abgabevorrichtung direkt nach erfolgter Montage in das Lager überführt und gelagert werden. Dabei ist das Lager insbesondere angefertigt, um mehr als eine montierte Abgabevorrichtung zu lagern.

Alternativ oder zusätzlich sind zur Lösung der genannten Aufgabe erfindungsgemäß die Merkmale des nebengeordneten, auf eine Abgabevorrichtung gerichteten Anspruchs vorgesehen. Insbesondere wird somit zur Lösung der genannten Aufgabe bei einer Abgabevorrichtung der eingangs beschriebenen Art erfindungsgemäß vorgeschlagen, dass die Abgabevorrichtung nach einem hierin beschriebenen Verfahren und/oder durch eine hierin beschriebene Montiervorrichtung hergestellt wird. Hierdurch kann eine Abgabevorrichtung besonders schnell und kostengünstig hergestellt werden.

Die Erfindung wird nun anhand von Ausführungsbeispielen näher beschrieben, ist jedoch nicht auf diese Ausführungsbeispiele beschränkt. Weitere Ausführungsbeispiele ergeben sich durch Kombination der Merkmale einzelner oder mehrerer Ansprüche untereinander und/oder mit einzelnen oder mehreren Merkmalen der Ausführungsbeispiele.

Es zeigen:
- Figur 1: eine erfindungsgemäße Montiervorrichtung, wobei eine montierte Abgabevorrichtung mit einem Transportsystem abtransportiert wird,
- Figur 2: die Montiervorrichtung aus Figur 1 und eine Inspektionsvorrichtung, wobei das erste Geräteteil durch die Geräteaufnahme positioniert ist und das zweite Geräteteil inspiziert wird,
- Figur 3: die Montiervorrichtung aus den vorherigen Figuren während der Überführung des zweiten Geräteteils von der ersten Handhabevorrichtung zur zweiten Handhabevorrichtung,
- Figur 4: die Montiervorrichtung aus den vorherigen Figuren, wobei das erste Geräteteil abströmseitig des zweiten Geräteteils mit dem zweiten Geräteteil verschraubt wird,
- Figur 5: eine alternative Ausführungsform eines Lagers für befüllte Behälter bei einer erfindungsgemäßen Montiervorrichtung,
- Figur 6: eine weitere alternative Ausführungsform eines Lagers für befüllte Behälter bei einer erfindungsgemäßen Montiervorrichtung,
- Figur 7: eine alternative Ausführungsform eines erfindungsgemäßen Verfahrens, wobei die montierte Abgabevorrichtung nach erfolgter Montage in ein Lager überführt ist.

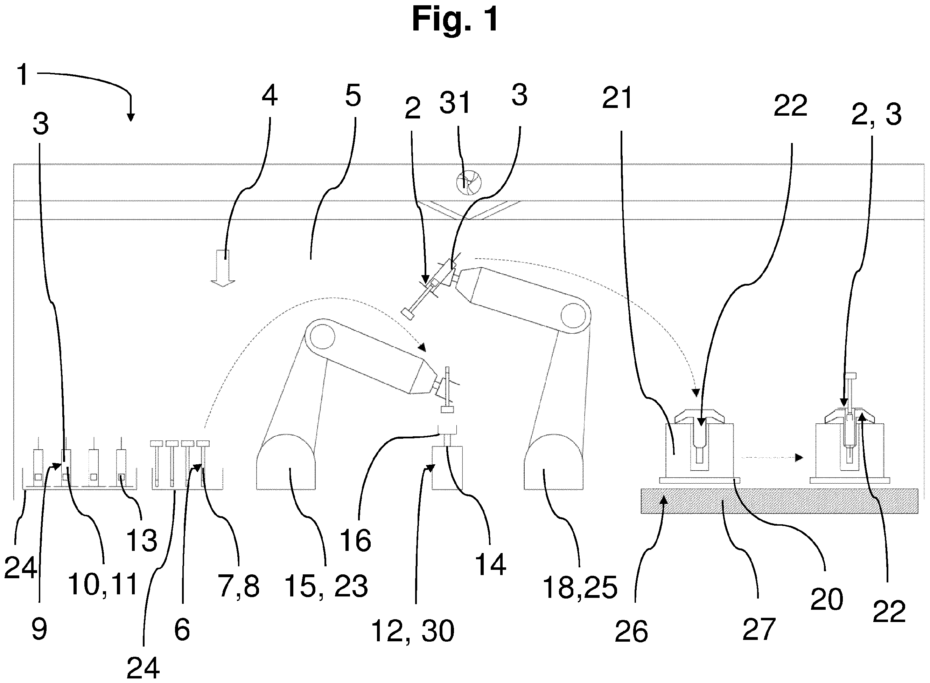

Die Figuren 1-4 zeigen ein Verfahren zur Montage einer Abgabevorrichtung 2 eines befüllten Behälters 3 in einem geschützten Arbeitsraum 5, wobei die Abgabevorrichtung 2 aus einem ersten Geräteteil 6, insbesondere einem zu einem Auslösen einer Abgabe eines Behälterinhalts aus dem Behälter 3 eingerichteten Betätigerteil 7, und einem zweiten Geräteteil 9, einem befüllten Behälterkörper 10, montiert wird. Das Verfahren umfasst folgende Verfahrensschritte: Durchströmung des Arbeitsraums 5 mit einer laminaren und/oder unidirektionalen Luftströmung 4; Positionierung des ersten und zweiten Geräteteils 6,9 in der Luftströmung 4, und Verbinden des ersten Geräteteils 6 mit dem zweiten Geräteteil 9 abströmseitig des zweiten Geräteteils 9. In dem hier gezeigten Verfahren wird ein als Abgabevorrichtung 2 ausgebildeter Sicherheitsspritze hergestellt.

Das in den Figuren 1-4 gezeigte Verfahren wird durch eine Montiervorrichtung 1 ausgeführt, wie sie nachfolgend beschrieben wird. Die Montiervorrichtung 1 hat einen geschützten, mit einer Luftströmung 4 durchströmten Arbeitsraum 5, wobei die Abgabevorrichtung 2 wenigstens ein erstes Geräteteil 6, das bereits genannte Betätigerteil 7, und ein zweites Geräteteil 9, den bereits genannten, befüllten Behälterkörper 10 (Spritzenkörper 11) hat. Die Luftströmung 4 wird durch eine Lüftungsanalage 31 generiert und aufrechterhalten. Ferner weist die Montiervorrichtung 1 eine Geräteaufnahme 12 auf, durch welche das erste Geräteteil 6 für die Montage positioniert wird. Ferner ist in den Figuren 1-4 zu sehen, dass die Montiervorrichtung 1 eine erste und eine zweite Handhabevorrichtung 15,18 aufweist.

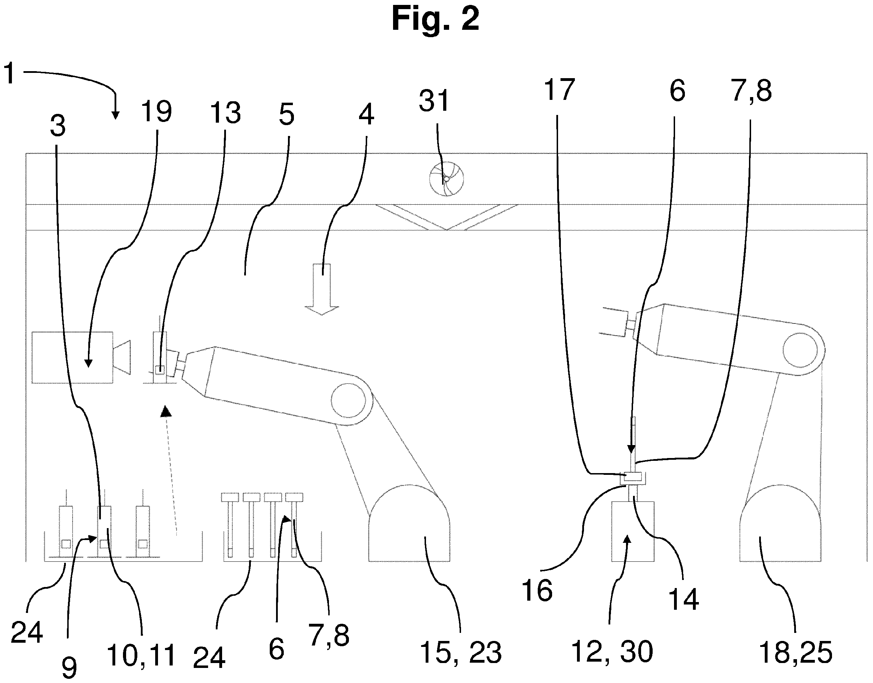

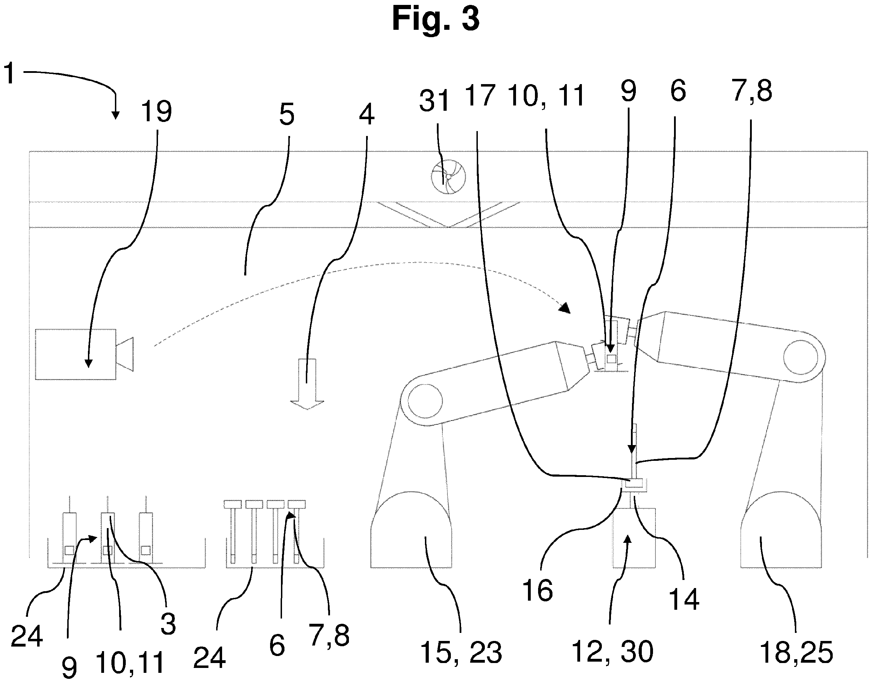

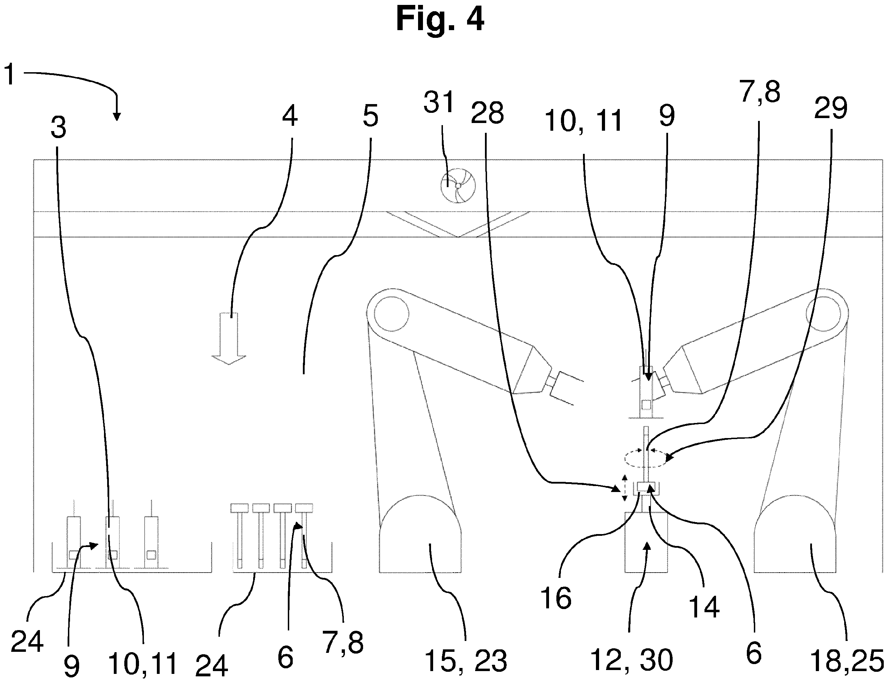

Die erste Handhabevorrichtung 15, welche als ein Greifroboter 23 ausgebildet ist, überführt das erste Geräteteil 6, welches eine Kolbenstange 8 hat, an die Geräteaufnahme 12, welche eine Verbindungsvorrichtung 30 ist. Die Geräteaufnahme 12, 30, insbesondere wie in Figur 4 zu erkennen ist, führt als Dreh-Hub-Vorrichtung 14 zum Einschrauben des ersten Geräteteils 6 in das zweite Geräteteil 9 abströmseitig des zweiten Geräteteils 9 eine Drehbewegung 29 und eine Hubbewegung 28 durch. Dabei greift die Greifvorrichtung 16 der Geräteaufnahme 12, 30 das erste Geräteteil 6 vorteilhaft an dessen Kopfende 17 (vgl. Figuren 2 und 3). Durch eine derartige Montage ist oberhalb des zweiten Geräteteils 9 insbesondere während des Einschraubens viel Platz im geschützten Arbeitsraum 5 eingespart. Generell kann durch die Montiervorrichtung 1 platzsparend der geschützte Arbeitsraum 5 ausgebildet sein und eine Luftströmung 4 einfacher und kostengünstiger erhalten sein.

Die erste Handhabevorrichtung 15, 23 übergibt später ein zweites Geräteteil 9, 10, 11 an die zweite Handhabevorrichtung 18, welchen nun das zweite Geräteteil 9, 10, 11 greift und zur Montage der Abgabevorrichtung 2 in der Luftströmung 4 positioniert (vgl. Figur 3). Auch die zweite Handhabevorrichtung 18 ist als ein Greifroboter 25 ausgebildet, welcher analog zu der Handhabevorrichtung 15, 23 in wenigstens drei Freiheitsgraden bewegbar und somit variabel einsetzbar ist.

Insbesondere aus den Figuren 3 und 4 ist zu erkennen, dass verfahrensmäßig das erste Geräteteil 6, 7, 8 abströmseitig des zweiten Geräteteils 9 verschraubt wird, wobei das Einschrauben des ersten Geräteteils 6, 7, 8 durch eine der Luftströmung 4 entgegengerichteten Drehbewegung der Geräteaufnahme 12 durchgeführt wird. Hierbei wird das erste Geräteteil 6, 7, 8 mit einem beweglichen Stopfen 13 des zweiten Geräteteils 9, 10, 11 verbunden, wodurch keine Flüssigkeit aus dem befüllten Behälterkörper 9, 10, 11 treten kann.

Bei dem gezeigten Verfahren werden ein Drehmoment und ein Anpressdruck während des abströmseitigen Einschraubens durch die Geräteaufnahme 12, 30 überwacht. Somit kann ein Überdrehen des ersten Geräteteils 6, 7, 8 beim Einschrauben verhindert werden.

Ferner gibt es bei der Montiervorrichtung 1 eine Inspektionsvorrichtung 19, welche die Ausrichtung des ersten und zweiten Geräteteils 6, 9 prüft (vgl. Figur 2), während diese durch die erste Handhabevorrichtung 15, 23 gegriffen sind. Somit kann vorteilhaft bereits früh während der Montage eine Qualitätskontrolle vorgenommen werden, um zu verhindern, das falsche, insbesondere nicht zueinander passende, Geräteteile montiert werden.

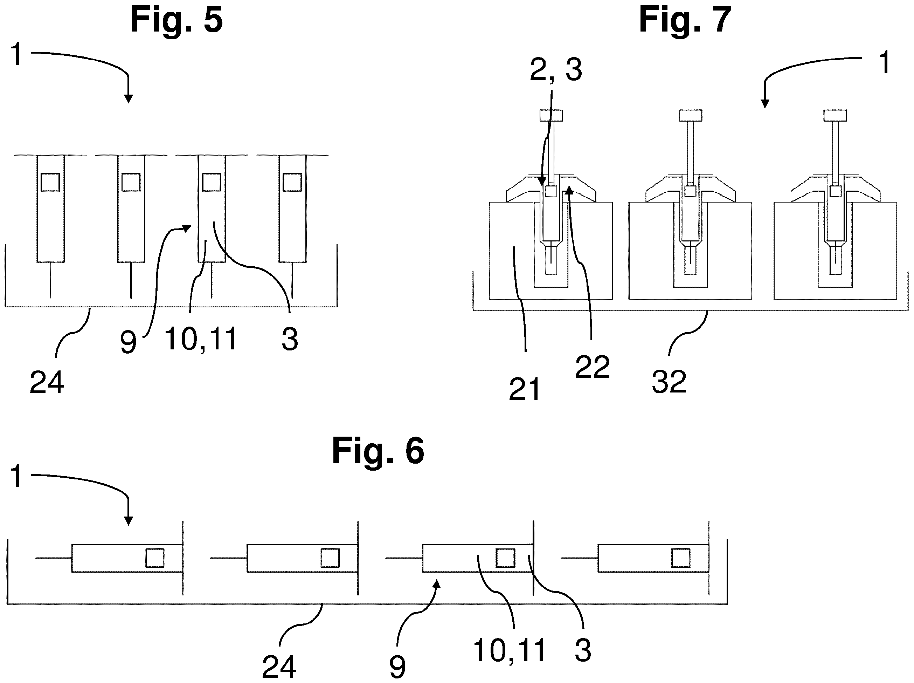

Die erste Handhabevorrichtung 15,23 nimmt das erste Geräteteil 6, 7, 8 bzw. zweite Geräteteil 9, 10, 11 aus einem Lager 24 (vgl. Figur 1). Das Lager 24 kann unterschiedlich ausgebildet sein, sodass insbesondere die zweiten Geräteteile 9, 10, 11 optimal positioniert werden können. Beispielsweise können die zweiten Geräteteile 9, 10, 11 mit ihrer spitzen, der Nadel umfassenden, Seite gegen die Luftströmung ragen (Figur 1), um 180° gedreht bezüglich der vorher genannten Ausführungsform (Figur 5) oder liegend positioniert sein (Figur 6). Während der Herausnahme durch die erste Handhabevorrichtung 15, 23, wie insbesondere in Figur 1 zu erkennen ist, übergibt die zweite Handhabevorrichtung 18, 25 eine bereits fertig montierte Abgabevorrichtung 2 an eine Transporteinheit 20 eines Transportsystems 26. Diese Übergabe erfolgt durch eine Aufwärtshubbewegung der zweiten Handhabevorrichtung 18, 25. Die Transporteinheit 20 ist magnetisch an eine StatorAnordnung 27 gekoppelt. Die Transporteinheit 20 umfasst ein passgenaues Formteil 21. Das Formteil 21 ist passgenau an ein drittes Geräteteil 22 (Case) der Sicherheitsspritze angepasst. Somit überführt die Handhabevorrichtung 18, 25 die Abgabevorrichtung 2 zu dem dritten Geräteteil 22, wodurch die Sicherheitsspritze final montiert wird.

Alternativ kann die montierte Abgabevorrichtung 2 durch die zweite Handhabevorrichtung 18, 25 an ein Lager 32 übergeben werden. Dieses Lager 32 kann, wie in Figur 7 dargestellt, ein passgenaues Formteil 21 haben. Das passgenaue Formteil 21 kann vor der Übergabe bereits das dritte Geräteteil 22 aufweisen, oder bei einer alternativen, nicht gezeigten Ausführungsform durch die Übergabe das dritte Geräteteil 22 mitsamt Abgabevorrichtung 2 erhalten.

Anschließend wird die Transporteinheit 20 mitsamt montierter Sicherheitsspritze durch magnetische Levitation auf der Statoranordnung 27 abtransportiert (vgl. Figur 1).

Die Erfindung schlägt allgemein Verfahren zur Montage einer Abgabevorrichtung 2 eines befüllten Behälters 3 in einem vorzugsweise geschützten Arbeitsraum 5, wobei die Abgabevorrichtung 2 aus wenigstens einem ersten Geräteteil 6, insbesondere einem zu einem Auslösen einer Abgabe eines Behälterinhalts aus dem Behälter 3 eingerichteten Betätigerteil 7, und einem zweiten Geräteteil 9, vorzugsweise einem befüllten Behälterkörper 10, montiert wird, vor, umfassend folgende Verfahrensschritte: Durchströmung des Arbeitsraums 5 mit einer vorzugsweise laminaren und/oder unidirektionalen Luftströmung 4; Positionierung des ersten und zweiten Geräteteils 6, 9 in der Luftströmung 4, und Verbinden des ersten Geräteteils 6 mit dem zweiten Geräteteil 9 abströmseitig des zweiten Geräteteils 9 (Figur 4).

### Bezugszeichenliste

- 1: Montiervorrichtung
- 2: Abgabevorrichtung
- 3: Behälter
- 4: Luftströmung
- 5: Arbeitsraum
- 6: erstes Geräteteil
- 7: Betätigerteil
- 8: Kolbenstange
- 9: zweites Geräteteil
- 10: Behälterkörper
- 11: Spritzenkörper
- 12: Geräteaufnahme
- 13: Stopfen
- 14: Dreh-Hub-Vorrichtung
- 15: erste Handhabevorrichtung
- 16: Greifvorrichtung von 12
- 17: Kopfende von 6
- 18: zweite Handhabevorrichtung
- 19: Inspektionsvorrichtung
- 20: Transporteinheit
- 21: Formteil von 20
- 22: drittes Geräteteil
- 23: erster Greifroboter
- 24: Lager
- 25: zweiter Greifroboter
- 26: Transportsystem
- 27: Stator-Anordnung
- 28: Hubbewegung
- 29: Drehbewegung
- 30: Verbindungsvorrichtung
- 31: Lüftungsanlage
- 32: Lager von 2 (nach Montage)

## Patentansprüche

1. Verfahren zur Montage einer Abgabevorrichtung (2) eines befüllten Behälters (3) in einem vorzugsweise geschützten Arbeitsraum (5), wobei die Abgabevorrichtung (2) aus wenigstens einem ersten Geräteteil (6), insbesondere einem zu einem Auslösen einer Abgabe eines Behälterinhalts aus dem Behälter (3) eingerichteten Betätigerteil (7), und einem zweiten Geräteteil (9), vorzugsweise einem befüllten Behälterkörper (10), montiert wird, umfassend folgende Verfahrensschritte: Durchströmung des Arbeitsraums (5) mit einer vorzugsweise lokal eine Hauptströmung definierende Luftströmung (4); Positionierung des ersten und zweiten Geräteteils (6, 9) in der Luftströmung (4), und Verbinden des ersten Geräteteils (6) mit dem zweiten Geräteteil (9) abströmseitig des zweiten Geräteteils (9).

2. Verfahren nach Anspruch 1, wobei das erste Geräteteil (6) eine Kolbenstange (8) (Plunger Rod) hat und/oder das zweite Geräteteil (9) einen Spritzenkörper (11) hat und/oder das erste Geräteteil (6) mit einem beweglichen Stopfen (13) des zweiten Geräteteils (9) verbunden wird.

3. Verfahren nach einem der vorangehenden Ansprüche, wobei eine Handhabevorrichtung (15), insbesondere ein Greifroboter (23), das erste und/oder das zweite Geräteteil (6, 9) aus einem Lager (24) entnimmt, wobei die Handhabevorrichtung (15) zur Positionierung des ersten Geräteteils (6) das erste Geräteteil (6) an eine Geräteaufnahme (12) überführt und/oder wobei die Handhabevorrichtung (15) zur Positionierung des zweiten Geräteteils (9) das zweite Geräteteil (9) an eine zweite Handhabevorrichtung (18), insbesondere ein zweiter Greifroboter (25), übergibt, wobei insbesondere das erste Geräteteil (6) durch die Geräteaufnahme (12) lösbar vorzugsweise an einem Kopfende (17) gegriffen wird.

4. Verfahren nach einem der vorhergehenden Ansprüche, wobei bei das erste Geräteteil (6) abströmseitig des zweiten Geräteteils (9) eingeführt, insbesondere eingeschraubt, wird, wobei vorzugsweise das Einführen, insbesondere Einschrauben, des ersten Geräteteils (6) durch eine der Luftströmung (4) entgegengerichtete Hubbewegung, insbesondere eine Dreh-Hub-Bewegung, der Geräteaufnahme (12) durchgeführt wird, und/oder wobei ein Drehmoment und/oder ein Anpressdruck während des abströmseitigen Verbindens insbesondere durch die Geräteaufnahme (12) überwacht wird.

5. Verfahren nach einem der vorhergehenden Ansprüche, wobei das zweite Geräteteil (9) durch die erste oder zweite Handhabevorrichtung (15, 18) zuströmseitig des ersten Geräteteils (6) positioniert wird und/oder wobei das zweite Geräteteil (9) durch die erste oder zweite Handhabevorrichtung (15, 18) gehalten und zur Positionierung ausgerichtet, insbesondere in einem Winkel von 180° gedreht, wird.

6. Verfahren nach einem der vorhergehenden Ansprüche, wobei vorzugsweise eine Eigenschaft/Merkmal, insbesondere Ausrichtung und/oder Farbe, des ersten und/oder des zweiten Geräteteils (6, 9) inspiziert wird, insbesondere wobei die Inspektion stattfindet, während das erste und/oder das zweite Geräteteil (6, 9) durch die erste Handhabevorrichtung (15, 18) gegriffen wird/ werden.

7. Verfahren nach einem der vorhergehenden Ansprüche, wobei die erste und/oder die zweite Handhabevorrichtung (15, 18) nach dem Verbinden eine Aufwärtshubbewegung durchführen und/oder die Abgabevorrichtung (2) in ein Formteil (21) und/oder ein Lager (32) und/oder ein drittes Geräteteil (Case) (22) und/oder auf ein Transportsystem (26), insbesondere umfassend eine magnetisch levitierte Transporteinheit (20), überführen.

8. Montiervorrichtung (1) zur Montage einer Abgabevorrichtung (2) eines befüllten Behälters (3) in einem vorzugsweise geschützten, mit einer Luftströmung (4) durchströmten Arbeitsraum (5), wobei die Abgabevorrichtung (2) wenigstens ein erstes Geräteteil (6), insbesondere ein zu einem Auslösen einer Abgabe eines Behälterinhalts aus dem Behälter (3) eingerichtetes Betätigerteil (7), und ein zweites Geräteteil (9), insbesondere einen befüllten Behälterkörper (10), umfasst, insbesondere zur Durchführung eines wie zuvor beanspruchten Verfahrens, umfassend: eine Geräteaufnahme (12) zur Positionierung des ersten Geräteteils (6); wenigstens eine Handhabevorrichtung (15, 18) zur Positionierung des zweiten Geräteteils (9) zuströmseitig des ersten Geräteteils (6); wenigstens eine Verbindungsvorrichtung (30), insbesondere Verschraubvorrichtung, zum Verbinden, insbesondere Verschrauben, des ersten Geräteteils (6) mit dem zweiten Geräteteil (9).

9. Montiervorrichtung (1) nach dem vorangehenden Anspruch, wobei das erste Geräteteil (7) eine Kolbenstange (8) (Plunger Rod) hat und/oder das zweite Geräteteil (9) einen Spritzenkörper (11) hat, und/oder wobei das erste Geräteteil (7) mit einem beweglichen Stopfen (13) des Behälters (3) verbindbar ist.

10. Montiervorrichtung (1) nach einem auf eine Montiervorrichtung (1) gerichteten, vorangehenden Anspruch, wobei die Geräteaufnahme (13) die Verbindungsvorrichtung (30) ist, insbesondere wobei die Geräteaufnahme (13) hubbewegbar und/oder dreh-hub-bewegbar ist, und/oder wobei die Geräteaufnahme (12) ein oder maximal zwei Freiheitsgrade hat.

11. Montiervorrichtung (1) nach einem auf eine Montiervorrichtung (1) gerichteten, vorangehenden Anspruch, wobei das erste Geräteteil (6) durch die wenigstens eine Handhabevorrichtung (15) an die Geräteaufnahme (12) überführbar ist, insbesondere wobei die Greifvorrichtung (16) das erste Geräteteil (6) an einem Kopfende (17) lösbar greift.

12. Montiervorrichtung (1) nach dem vorherigen Anspruch, wobei die Gerätaufnahme (12) eine Greifvorrichtung (16) aufweist, insbesondere wobei die Greifvorrichtung (16) eine Dreh-Hub-Vorrichtung (14) ist, und/oder wobei die Geräteaufnahme (12) zur Überwachung eines Drehmoments und/oder eines Anpressdrucks eingerichtet ist.

13. Montiervorrichtung (1) nach einem auf eine Montiervorrichtung (1) gerichteten, vorangehenden Anspruch, wobei die Montiervorrichtung (1) wenigstens eine erste und eine zweite Handhabevorrichtung aufweist, wobei wenigstens ein Geräteteil (6, 9), vorzugsweise das zweite Geräteteil (9), durch die erste Handhabevorrichtung (15) an die zweite Handhabevorrichtung (18) übergebbar ist, und/oder wobei das erste und/oder das zweite Geräteteil (6, 9) und/oder die montierte Abgabevorrichtung (2) durch die erste und/oder zweite Handhabevorrichtung (15, 18) in wenigstens drei Freiheitsgraden bewegbar und/oder positionierbar ist.

14. Montiervorrichtung (1) nach einem auf eine Montiervorrichtung (1) gerichteten, vorangehenden Anspruch, wobei die Montiervorrichtung (1) eine Inspektionsvorrichtung (19), insbesondere zur Inspektion einer Eigenschaft, insbesondere einer Ausrichtung und/oder Farbe des ersten und/oder zweiten Geräteteils (6, 9), aufweist.

15. Montiervorrichtung (1) nach einem auf eine Montiervorrichtung (1) gerichteten, vorangehenden Anspruch, wobei der geschützte Arbeitsraum (5) eine Lüftungsanlage und/oder Luftöffnungen zur Ausbildung der Luftströmung (4) aufweist, und/oder wobei die montierte Abgabevorrichtung (2) durch die erste und/oder zweite Handhabevorrichtung (15, 18) an ein vorzugsweise auf einer, vorzugsweise magnetisch levitierbaren, Transporteinheit (20) befindliches, passgenaues Formteil (21) übergebbar ist, insbesondere wobei sich an dem passgenauen Formteil (21) ein drittes Geräteteil (Case) (22) befindet, in welches die Abgabevorrichtung (2) eingesetzt wird, und/oder wobei das passgenaue Formteil (21) in einem Lager (32) positioniert ist.

16. Abgabevorrichtung (2), hergestellt nach einem vorhergehend beanspruchten Verfahren und/oder mit einer vorhergehend beanspruchten Montiervorrichtung (1).
